# EUROPÄISCHE PATENTSCHRIFT

(11) **EP 3 085 320 B1**
(45) Veröffentlichungstag und Bekanntmachung des Hinweises auf die Patenterteilung: **20.04.2022**
(21) Anmeldenummer: 16157414.0
(22) Anmeldetag: 25.02.2016
(51) Int. Cl.: A61B 17/70, A61B 17/86

(54) **MONOAXIALKNOCHENSCHRAUBE**
MONOAXIAL BONE SCREW
VIS A OS MONO-AXIALE

(30) Priorität: 25.04.2015 DE 202015003062 U
(43) Veröffentlichungstag der Anmeldung: 26.10.2016
(73) Patentinhaber: Silony Medical International AG, 8500 Frauenfeld (CH)
(72) Erfinder: Heuer, Frank, 70794 Filderstadt (DE); Trautwein, Frank, Thilo, 70794 Filderstadt (DE); Tautz, Katharina, 70192 Stuttgart (DE)
(74) Vertreter: DREISS Patentanwälte PartG mbB

(56) Entgegenhaltungen:
- WO-A1-2014/138736
- US-A1- 2006 111 713
- US-A1- 2007 161 985
- US-A1- 2013 079 830
- US-A1- 2014 142 633

## Beschreibung

Die Erfindung betrifft eine Monoaxialknochenschraube mit einem ein Außengewinde aufweisenden Schaft und einem daran proximal und einstückig anschließenden Gabelkopf, der also starr mit dem Schaft ausgebildet ist, wobei der Gabelkopf in einer Seitenansicht U-förmig ist und zwischen die U-Form bildenden Schenkeln eine Aufnahmeöffnung für ein Korrekturelement, insbesondere einen Korrekturstab, aufweist, wobei eine Längsrichtung und eine hierzu radiale Richtung gegeben ist, wobei die Aufnahmeöffnung in dem Gabelkopf ein distales Ende benachbart zu dem Schaft und ein hiervon in Längsrichtung abgewandtes offenes proximales Ende aufweist, in welches eine Klemmschraube für das Korrekturelement einschraubbar ist, wobei die Schenkel des Gabelkopfs hierfür ein Innengewinde tragen, wobei im Bereich des distalen Endes der Aufnahmeöffnung eine Werkzeugansetzstelle in Form einer zentrischen und in Längsrichtung erstreckten Vertiefung zur komplementären Aufnahme eines Werkzeugs ausgebildet ist, derart, dass die Schraube mittels des in die Vertiefung eingesetzten Werkzeugs um die Längsrichtung drehbar und hierdurch in den Knochen einschraubbar ist, wobei die Schenkel einen radial äußeren Umfangsbereich aufweisen, der in einer Ebene orthogonal zur Längsrichtung des Gabelkopfs bogenförmig, insbesondere kreisbogenförmig gekrümmt ausgebildet ist und in dem zum Ergreifen des Gabelkopfs mittels eines Handhabungsinstruments jeweils wenigstens eine Haltenut ausgebildet ist. Eine derartige Monoaxialknochenschraube ist bekannt aus US 2013/0079830 A1.

Da der Gabelkopf bei Monoaxialknochenschrauben mit dem Schaft starr verbunden ist, wurden Monoaxialknochenschrauben auch mittels eines an den Schenkeln des Gabelkopfs angreifenden Einschraubinstruments ergriffen und in den Knochen des Patienten eingeschraubt.

Der vorliegenden Erfindung liegt die Aufgabe zugrunde, die Handhabbarkeit einer Monoaxialknochenschraube der genannten Art zu verbessern.

Diese Aufgabe wird durch eine Monoaxialknochenschraube mit den Merkmalen des Anspruchs 1 gelöst.

Es erweist sich von der Kinematik des Einschraubens her als vorteilhaft, wenn das Drehmoment mittels eines hierfür verwandten Instruments so nahe wie möglich am Schaft in die Knochenschraube eingeleitet wird. Dadurch dass die Werkzeugansetzstelle im Bereich des distalen Endes der Aufnahmeöffnung des Gabelkopfs vorgesehen wird, kann dies erreicht werden. Ein weiterer Vorteil ist, dass zum Einschrauben der Monoaxialknochenschraube dasselbe Instrument wie zum Einschrauben einer Polyaxialknochenschraube verwendet werden kann, bei denen typischerweise am distalen Ende eines im Gabelkopfs verschwenkbaren Kopfs des Schafts eine Werkzeugansetzstelle vorgesehen ist.

In vorteilhafter Weise wird vorgeschlagen, dass die Werkzeugansetzstelle als Imbus- oder Torx-Werzeugansetzstelle ausgebildet ist.

Des Weiteren wird vorgeschlagen, dass die Werkzeugansetzstelle im Bereich des distalen Endes der Aufnahmeöffnung mit einer in Längsrichtung verlaufenden Öffnung in dem Schaft kommuniziert, wobei diese in Längsrichtung verlaufenden Öffnung in der Werkzeugansetzstelle proximal ausmündet und im Bereich des Schafts über Queröffnungen in radialer Richtung ausmündet sowie in Längsrichtung distal ausmündet.

Zusätzlich zu der Haltenut erweist es sich als vorteilhaft, wenn in dem radial äußeren Umfangsbereich zum Ergreifen des Gabelkopfs mittels eines Handhabungsinstruments jeweils wenigstens eine in radialer Richtung erstreckte, vorzugsweise zylindrische Haltevertiefung ausgebildet ist.

Gegenstand der Erfindung ist auch eine Sachgesamtheit aus einer Monoaxialschraube nach einem oder mehreren der vorstehenden Ansprüche und einer Polyaxialschraube mit einem gegenüber dem Schaft polyaxial verschwenkbaren Gabelkopf, ebenfalls mit einer Aufnahmeöffnung für ein Korrekturelement, dadurch gekennzeichnet, dass die Werkzeugansetzstelle im Bereich des distalen Endes der Aufnahmeöffnung der Monoaxialschraube identisch zu einer Werkzeugansetzstelle im Bereich des distalen Endes der Aufnahmeöffnung der Polyaxialschraube ausgebildet ist. Weiter wird eine solche Sachgesamtheit mit einem Werkzeug für beide Schrauben beansprucht.

Weitere Merkmale, Einzelheiten und Vorteile der Erfindung ergeben sich aus den beigefügten Schutzansprüchen und aus der zeichnerischen Darstellung und nachfolgenden Beschreibung einer vorteilhaften Ausführungsform der vorliegenden Erfindung.

In der Zeichnung zeigt:
- Figur 1: eine Seitenansicht einer erfindungsgemäßen Monoaxialknochenschraube;

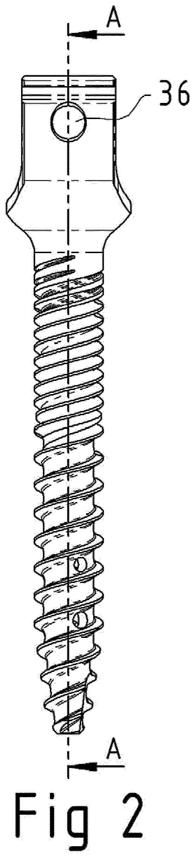

- Figur 2: eine andere Seitenansicht der Knochenschraube nach Figur 1;

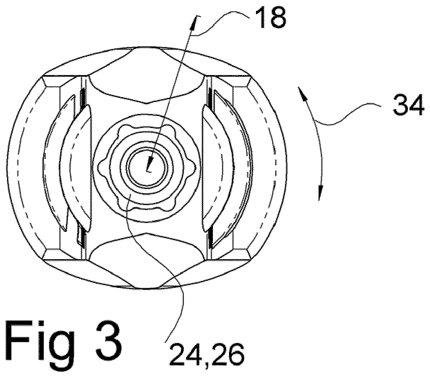

- Figur 3: eine Draufsicht auf einen Gabelkopf der Knochenschraube nach Figur 1;

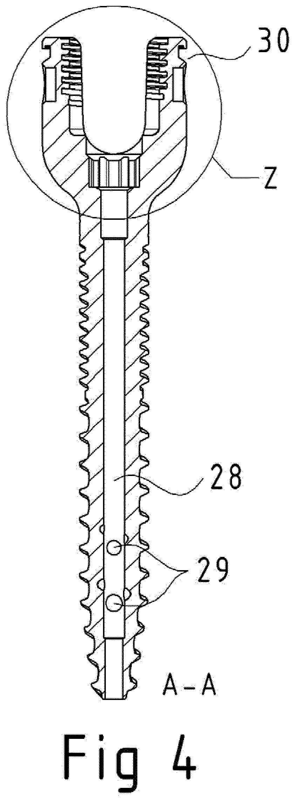

- Figur 4: eine Schnittansicht mit Schnittebene A-A gemäß Figur 2;
- Figur 5: eine Einzelheit Z der Figur 4;

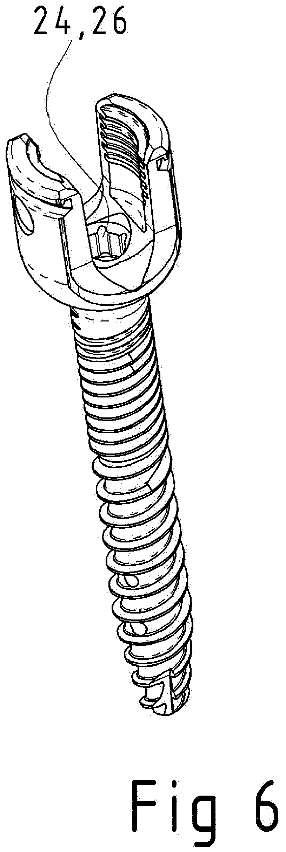

- Figur 6: eine perspektivische Ansicht der Knochenschraube nach Figur 1; und
- Figur 7: eine Einzelheit der Figur 5.

Die Figuren zeigen eine insgesamt mit dem Bezugszeichen 2 bezeichnete Monoaxialknochenschraube. Die Schraube umfasst einen Schaft 4 mit einem Außengewinde 6 und einen Gabelkopf 8, der sich in proximaler Richtung und einstückig an den Schaft 4 anschließt. Der Gabelkopf 8 ist also starr und damit undrehbar mit dem Schaft 4 ausgebildet.

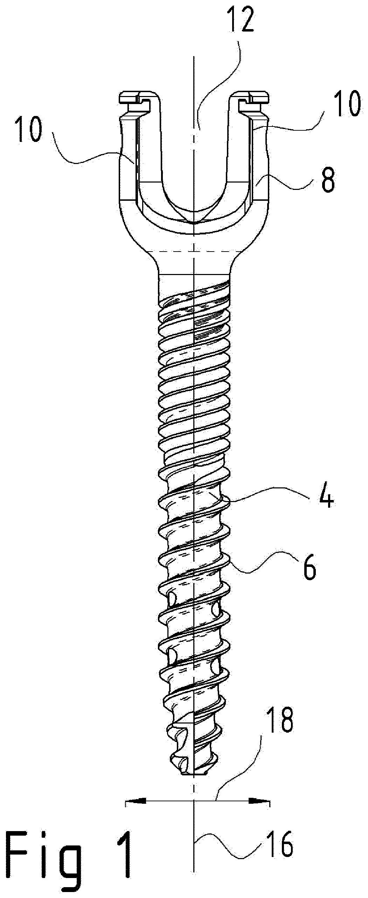

Der Gabelkopf 8 umfasst in an sich bekannter Weise zwei Schenkel 10, die dem Gabelkopf 8 in der Seitenansicht der Figur 1 betrachtet eine U-Form verleihen. Die Schenkel 10 begrenzen zwischen sich eine Aufnahmeöffnung 12 für ein Korrekturelement, insbesondere einen in den Figuren nicht dargestellten Korrekturstab, der sich im eingesetzten Betriebszustand senkrecht zu der Zeichnungsebene der Figur 1 erstrecken würde. Dieses Korrekturelement wird durch Einschrauben einer ebenfalls nicht dargestellten an sich bekannten Klemmschraube in ein Innengewinde 14 fixiert, welches radial innen an den Schenkeln 10 ausgebildet ist. Aus der Geometrie der Monoaxialknochenschraube 2 ergeben sich eine Längsrichtung oder -achse 16 und eine radiale Richtung 18.

Die Aufnahmeöffnung 12 umfasst ein distales Ende 20, welches auch den Scheitel der U-Form bildet, und ein hiervon in der Längsrichtung 16 abgewandtes proximales Ende 22, in welches die erwähnte Klemmschraube einschraubbar ist. Erfindungsgemäß ist im Bereich des distalen Endes 20 der Aufnahmeöffnung 12 eine Werkzeugansetzstelle 24, vorzugsweise eine Imbus- oder Torx-Werkzeugansetzstelle ausgebildet. Die Werkzeugansetzstelle 24 ist in Form einer zentrisch angeordneten und in der Längsrichtung 16 in den Schaft 4 hineinerstreckten Vertiefung 26 ausgebildet. Sie dient der komplementären Aufnahme eines nicht dargestellten Schraubwerkzeugs. Dadurch dass das Schraubwerkzeug in die Vertiefung 26 exakt zentrisch und sehr nahe am Schaft 4 eingesteckt werden kann, lässt sich handgeführt mit feinfühliger Rückwirkung auf den Chirurgen ein Drehmoment einleiten, um den Schraubenschaft in den Knochen einzudrehen.

Der Schaft 4 ist vorliegend kanüliert ausgebildet und weist eine in Längsrichtung 16 verlaufend Öffnung 28 auf, die in der Werkzeugansetzstelle 24 proximal ausmündet. Sie mündet auch über Queröffnungen 29 radial und in Längsrichtung 18 distal aus.

Weiter ist in einem radial äußeren Umfangsbereich 30 der Schenkel 10 des Gabelkopfs 8 je eine Haltenut 32 ausgebildet, die sich in einer Umfangsrichtung 34 des Gabelkopfs 8 und einer Ebene orthogonal zur Längsrichtung 16 erstreckt. Diese Haltenut ist wie eingangs beschrieben ausgebildet.

Des Weiteren ist in dem jeweiligen radial äußeren Umfangsbereich 30 je eine in radialer Richtung 18 erstreckte vorzugsweise zylindrische Vertiefung 36 ausgebildet, die ebenfalls zum Ergreifen der Monoaxialknochenschraube mittels eines Handhabungsinstruments dient.

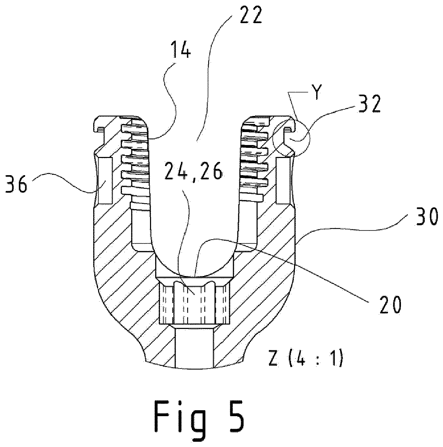

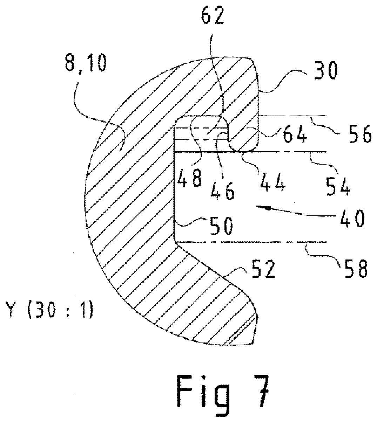

Figur 7 zeigt vergrößert die Haltenut 32 von Figur 5, wobei die Zeichnungsebene in einer die Längsrichtung 16 des Gabelkopfs 8 einschließenden Schnittebene dargestellt ist. Ausgehend von der Oberfläche des Umfangsbereichs 30 im Bereich des proximalen Endes des Gabelkopfs 8 wird die Haltenut 32 begrenzt von einem ersten bis fünften Flankenabschnitt 44, 46, 48, 50 bzw. 52. Der erste Flankenabschnitt 44 erstreckt sich nach radial innen und verläuft in einer Ebene 54 orthogonal zur Längsrichtung 16 des Gabelkopfs 8. Daran anschließend erstreckt sich der zweite Flankenabschnitt 46 in proximaler Richtung und verläuft konzentrisch zur Längsrichtung 16 des Gabelkopfs 8. Daran anschließend erstreckt sich der dritte Flankenabschnitt 48 nach radial innen und verläuft in einer Ebene 56 orthogonal zur Längsrichtung 16 des Gabelkopfs 8. Daran anschließend erstreckt sich der vierte Flankenabschnitt 50 in distaler Richtung und verläuft konzentrisch zur Längsrichtung 16 des Gabelkopfs 8. Er erstreckt sich dabei in distaler Richtung bis unter den ersten Flankenabschnitt 44. Anschließend an das distale Ende des vierten Flankenabschnitts 50 erstreckt sich der fünfte Flankenabschnitt 52 mit einer Komponente nach radial außen und verläuft im beispielhaft dargestellten Fall in einem Winkel zu einer Ebene 58 orthogonal zur Längsrichtung 16 des Gabelkopfs 8, also distal geneigt zur Horizontalen.

Die Haltenut 32 bildet daher einen in radialer Richtung 18 hintergreifbaren Bereich 62. Dieser Bereich 62 ist radial außen begrenzt von dem zweiten Flankenabschnitt 46. Man erkennt einen in der Schnittansicht der Figur 7 ungefähr rechteckförmigen Bund 64 zwischen dem zweiten Flankenabschnitt 46 und der äußeren Oberfläche des Umfangsbereichs 30, der in distaler Richtung über den dritten Flankenabschnitt 48 in Längsrichtung 16 vorsteht.

## Patentansprüche

1. Monoaxialknochenschraube (2) mit einem ein Außengewinde aufweisenden Schaft (4) und einem daran proximal und einstückig anschließenden Gabelkopf (8), der also starr mit dem Schaft (4) ausgebildet ist, wobei der Gabelkopf (8) in einer Seitenansicht U-förmig ist und zwischen die U-Form bildenden Schenkeln (10) eine Aufnahmeöffnung (12) für ein Korrekturelement, insbesondere einen Korrekturstab, aufweist, wobei eine Längsrichtung (16) und eine hierzu radiale Richtung (18) gegeben ist, wobei die Aufnahmeöffnung (12) in dem Gabelkopf (8) ein distales Ende (20) benachbart zu dem Schaft (4) und ein hiervon in Längsrichtung (16) abgewandtes offenes proximales Ende (22) aufweist, in welches eine Klemmschraube für das Korrekturelement einschraubbar ist, wobei die Schenkel (10) des Gabelkopfs (8) hierfür ein Innengewinde (14) tragen, wobei im Bereich des distalen Endes (20) der Aufnahmeöffnung (12) eine Werkzeugansetzstelle (24) in Form einer zentrischen und in Längsrichtung (16) erstreckten Vertiefung (26) zur komplementären Aufnahme eines Werkzeugs ausgebildet ist, derart, dass die Schraube mittels des in die Vertiefung eingesetzten Werkzeugs um die Längsrichtung (16) drehbar und hierdurch in den Knochen einschraubbar ist, wobei die Schenkel (10) einen radial äußeren Umfangsbereich (30) aufweisen, der in einer Ebene orthogonal zur Längsrichtung des Gabelkopfs bogenförmig, insbesondere kreisbogenförmig gekrümmt ausgebildet ist und in dem zum Ergreifen des Gabelkopfs (8) mittels eines Handhabungsinstruments jeweils wenigstens eine Haltenut (32) ausgebildet ist, **dadurch gekennzeichnet, dass** sich die Haltenut (32) in einer Umfangsrichtung (34) des Gabelkopfs (8) und in einer Ebene orthogonal zur Längsrichtung (16) des Gabelkopfs (8) erstreckt, und dass die Haltenut (32) in einer die Längsrichtung (16) einschließenden gedachten Schnittebene betrachtet ausgehend von einer Oberfläche des Umfangsbereichs (30) begrenzt ist
- von einem ersten nach radial innen erstreckten und in einer Ebene (54) orthogonal zur Längsrichtung (16) des Gabelkopfs (8) verlaufenden Flankenabschnitt (44),
- daran anschließend von einem zweiten in proximaler Richtung erstreckten und konzentrisch zur Längsrichtung (16) verlaufenden Flankenabschnitt (46),
- daran anschließend von einem dritten nach radial innen erstreckten und in einer Ebene (56) orthogonal zur Längsrichtung (16) des Gabelkopfs (8) verlaufenden Flankenabschnitt (48),
- daran anschließend von einem vierten in distaler Richtung erstreckten und konzentrisch zur Längsrichtung (16) verlaufenden Flankenabschnitt (50), der sich in distaler Richtung bis unter den ersten Flankenabschnitt (44) erstreckt, und
- daran anschließend von einem fünften mit einer Komponente nach radial außen erstreckten und in einem Winkel hierzu distal geneigt verlaufenden Flankenabschnitt (52).

2. Monoaxialknochenschraube (2) nach Anspruch 1, **dadurch gekennzeichnet, dass** die Werkzeugansetzstelle (24) als Inbus oder Torx-Werkzeugansetzstelle ausgebildet ist.

3. Monoaxialknochenschraube (2) nach Anspruch 1 oder 2, **dadurch gekennzeichnet, dass** die Werkzeugansetzstelle (24) im Bereich des distalen Endes (20) der Aufnahmeöffnung (12) mit einer in Längsrichtung (16) verlaufenden Öffnung (28) in dem Schaft (4) kommuniziert, wobei diese in Längsrichtung (16) verlaufenden Öffnung (28) in der Werkzeugansetzstelle (24) proximal ausmündet und im Bereich des Schafts (4) über Queröffnungen (29) in radialer Richtung (18) sowie in Längsrichtung (16) distal ausmündet.

4. Monoaxialknochenschraube (2) nach einem oder mehreren der vorstehenden Ansprüche, **dadurch gekennzeichnet, dass** in dem radial äußeren Umfangsbereich (30) zum Ergreifen des Gabelkopfs (8) mittels eines Handhabungsinstruments jeweils wenigstens eine in radialer Richtung (18) erstreckte, vorzugsweise zylindrische Haltevertiefung (36) ausgebildet ist.

5. Sachgesamtheit aus einer Monoaxialschraube (2) nach einem oder mehreren der vorstehenden Ansprüche und einer Polyaxialschraube mit einem gegenüber dem Schaft polyaxial verschwenkbaren Gabelkopf, ebenfalls mit einer Aufnahmeöffnung für ein Korrekturelement, **dadurch gekennzeichnet, dass** die Werkzeugansetzstelle im Bereich des distalen Endes der Aufnahmeöffnung der Monoaxialschraube identisch zu einer Werkzeugansetzstelle im Bereich des distalen Endes der Aufnahmeöffnung der Polyaxialschraube ausgebildet ist.

6. Sachgesamtheit nach Anspruch 5, weiter umfassend ein Werkzeug mit einem komplementär zu den beiden Werkzeugansetzstellen ausgebildeten Einsteckende, so dass dieses Werkzeug in beide Werkzeugansetzstellen einsteckbar ist.

## Claims

1. Monoaxial bone screw (2) with a shank (4) having an external thread and, proximally and integrally adjoining this, a fork head (8) which is thus formed rigidly with the shank (4), wherein the fork head (8) is U-shaped in side view and, between the legs (10) forming the U-shape, has a receiving opening (12) for a correction element, in particular a correction rod, wherein there is a longitudinal direction (16) and a radial direction (18), wherein the receiving opening (12) in the fork head (8) has a distal end (20) adjacent to the shank (4) and an open proximal end (22) which faces away from this in the longitudinal direction (16) and in which a clamping screw for the correction element can be screwed, wherein for this, the legs (10) of the fork head (8) have an internal thread (14), wherein in the region of the distal end (20) of the receiving opening (12), a tool application point (24) is formed in the form of a central depression (26) extending in the longitudinal direction (16) for complementarily receiving the tool, such that the screw can be turned about the longitudinal direction (16) by means of the tool inserted in the depression and thereby screwed into the bone, wherein the legs (10) have a radially outer peripheral region (30) which is configured so as to be arcuate, in particular curved in the form of a circle arc, in a plane orthogonal to the longitudinal direction of the fork head and in which at least one retaining groove (32) is formed for gripping the fork head (8) by means of a handling instrument, **characterised in that** the retaining groove (32) extends in a peripheral direction (34) of the fork head (8) and in a plane orthogonal to the longitudinal direction (16) of the fork head (8), and the retaining groove (32), viewed in a theoretical section plane enclosing the longitudinal direction (16), starting from a surface of the peripheral region (30), is delimited by
- a first flank portion (44) extending radially inward and running in a plane (54) orthogonal to the longitudinal direction (16) of the fork head (8),
- adjoining this, a second flank portion (46) extending in the proximal direction and running concentrically to the longitudinal direction (16),
- adjoining this, a third flank portion (48) extending radially inward and running in a plane (56) orthogonal to the longitudinal direction (16) of the fork head (8),
- adjoining this, a fourth flank portion (50) extending in the distal direction and running concentrically to the longitudinal direction (16), which flank portion extends in the distal direction to below the first flank portion (44), and
- adjoining this, a fifth flank portion (52) with a component extending radially outward and running distally sloping at an angle thereto.

2. Monoaxial bone screw (2) according to claim 1, **characterised in that** the tool application point (24) is configured as a hexagonal socket or Torx tool application point.

3. Monoaxial bone screw (2) according to claim 1 or 2, **characterised in that** the tool application point (24) in the region of the distal end (20) of the receiving opening (12) communicates with an opening (28) in the shank (4) running in the longitudinal direction (16), wherein this opening (28) running in the longitudinal direction (16) opens proximally into the tool application point (24) and opens distally in the region of the shank (4) via transverse openings (29) in the radial direction (18) and in the longitudinal direction (16) .

4. Monoaxial bone screw (2) according to one or more of the preceding claims, **characterised in that** in the radially outer peripheral region (30), for gripping the fork head (8) by means of a handling instrument, in each case at least one preferably cylindrical retaining depression (36) is formed extending in the radial direction (18).

5. Assembly of a monoaxial screw (2) according to one or more of the preceding claims and a polyaxial screw with a fork head which is polyaxially pivotable relative to the shank and also has a receiving opening for a correction element, **characterised in that** the tool application point in the region of the distal end of the receiving opening of the monoaxial screw is identical to a tool application point in the region of the distal end of the receiving opening of the polyaxial screw.

6. Assembly according to claim 5, furthermore comprising a tool with an insertion end formed complementarily to the tool application points so that said tool can be inserted in both tool application points.

## Revendications

1. Vis à os monoaxiale (2) comprenant une tige (4) ayant un filetage extérieur ainsi qu'une tête fourchue (8) se raccordant de façon proximale et d'un seul tenant, qui est donc conçue de manière rigide avec ladite tige (4), dans laquelle, vue de côté, la tête fourchue (8) présente une forme en U et comprend une ouverture de réception (12) pour un élément de correction, en particulier une tige de correction, entre des branches (10) formant la forme en U, une direction longitudinale (16) et une direction (18) radiale à celle-ci étant définies, dans laquelle ladite ouverture de réception (12) dans la tête fourchue (8) présente une extrémité distale (20) adjacente à la tige (4) ainsi qu'une extrémité proximale ouverte (22) qui montre dans la direction longitudinale (16) opposée à celle-ci et dans laquelle peut être vissée une vis de serrage pour ledit élément de correction, dans laquelle les branches (10) de la tête fourchue (8) présentent un filetage intérieur (14) à cette fin, dans laquelle un point d'application d'outil (24) sous la forme d'un creux (26) central et s'étendant dans la direction longitudinale (16) et destiné à recevoir de manière complémentaire un outil est formé au niveau de l'extrémité distale (20) de l'ouverture de réception (12), de telle sorte que la vis peut être tournée autour de la direction longitudinale (16) au moyen de l'outil inséré dans le creux et peut ainsi être vissée dans l'os, dans laquelle les branches (10) présentent une zone périphérique radialement extérieure (30) qui est courbée en arc, en particulier en arc de cercle, dans un plan orthogonal à la direction longitudinale de la tête fourchue et dans laquelle respectivement au moins une nervure de retenue (32) est formée pour saisir la tête fourchue (8) au moyen d'un instrument de manipulation, **caractérisée par le fait que** la rainure de retenue (32) s'étend dans une direction circonférentielle (34) de la tête fourchue (8) et dans un plan orthogonal à la direction longitudinale (16) de la tête fourchue (8), et que la rainure de retenue (32), vue dans un plan de coupe imaginaire incluant la direction longitudinale (16), est délimitée à partir d'une surface de la zone périphérique (30)
- par une première section de flanc (44) qui s'étend radialement vers l'intérieur et dans un plan (54) orthogonal à la direction longitudinale (16) de la tête fourchue (8),
- par une deuxième section de flanc (46) contiguë à cette dernière, qui s'étend dans la direction proximale (28) et de façon concentrique à la direction longitudinale (16),
- par une troisième section de flanc (48) contiguë à cette dernière, qui s'étend radialement vers l'intérieur et dans un plan (56) orthogonal à la direction longitudinale (16) de la tête fourchue (8),
- par une quatrième section de flanc (50) contiguë à cette dernière, qui s'étend dans la direction distale et de manière concentrique par rapport à la direction longitudinale (16) et qui s'étend dans la direction distale jusqu'au-dessous de la première section de flanc (44), et
- par une cinquième section de flanc (52) contiguë à cette dernière, qui s'étend radialement vers l'extérieur avec un composant et en étant inclinée de manière distale à un angle.

2. Vis à os monoaxiale (2) selon la revendication 1, **caractérisée par le fait que** le point d'application d'outil (24) est réalisé sous la forme d'un point d'application d'outil Allen ou Torx.

3. Vis à os monoaxiale (2) selon la revendication 1 ou 2, **caractérisée par le fait que** le point d'application d'outil (24) au niveau de l'extrémité distale (20) de l'ouverture de réception (12) est en communication avec une ouverture (28) dans la tige (4), qui s'étend dans la direction longitudinale (16), dans laquelle cette ouverture (28) s'étendant dans la direction longitudinale (16) débouche de façon proximale sur le point d'application d'outil (24) et de façon distale au niveau de la tige (4) via des ouvertures transversales (29) dans la direction radiale (18) et dans la direction longitudinale (16).

4. Vis à os monoaxiale (2) selon l'une ou plusieurs des revendications précédentes, **caractérisée par le fait que** respectivement au moins un creux de retenue (36) de préférence cylindrique qui s'étend dans la direction radiale (18) est ménagé dans la zone périphérique radialement extérieure (30) pour la préhension de la tête fourchue (8) au moyen d'un instrument de manipulation.

5. Ensemble matériel se composant d'une vis monoaxiale (2) selon l'une ou plusieurs des revendications précédentes et d'une vis polyaxiale ayant une tête fourchue apte à être pivotée de manière polyaxiale par rapport à la tige, ayant, elle aussi, une ouverture de réception pour un élément de correction, **caractérisé par le fait que** le point d'application d'outil au niveau de l'extrémité distale de l'ouverture de réception de la vis monoaxiale est réalisé de manière identique à un point d'application d'outil au niveau de l'extrémité distale de l'ouverture de réception de la vis polyaxiale.

6. Ensemble matériel selon la revendication 5, comprenant en outre un outil ayant une extrémité d'insertion réalisée de manière complémentaire aux deux points d'application d'outil, de sorte que cet outil peut être inséré dans les deux points d'application d'outil.
